# EUROPEAN PATENT APPLICATION

(11) **EP 2 664 327 A1**
(43) Date of publication of application: **20.11.2013**
(21) Application number: 12003791.6
(22) Date of filing: 14.05.2012
(51) Int. Cl.: A61K 31/137, A61K 31/17, A61P 33/14

(54) **Topical pharmaceutical compositions comprising terbinafide and urea**

(71) Applicant: Almirall S.A., 08022 Barcelona (ES)
(72) Inventor: Willers, Christoph, 21465 Reinbek (DE); Herbig, Michael, 21465 Reinbek (DE); Mallwitz, Henning, 21465 Reinbek (DE); Gorissen, Sascha, 21465 Reinbek (DE); Fielhauer, Sabine, 21465 Reinbek (DE); Evers, Dirk-Heinrich, 21465 Reinbek (DE)
(74) Representative: polypatent

(57) **Abstract**

The invention relates to topical pharmaceutical compositions comprising terbinafine and urea, and the use of these compositions in the treatment of topical fungal diseases, such as onychomycosis. The compositions provided are stable and show strong nail hydration and penetration.

## Description

### FIELD OF THE INVENTION

The present invention relates to topical pharmaceutical compositions comprising terbinafine and urea, and the use of these compositions In the treatment of topical fungal diseases, such as onychomycosis. The compositions provided are stable and show strong nail hydration and penetration.

### BACKGROUND OF THE INVENTION

Onychomycosis, also known as tinea unguium, is a fungal infection of the nails, having a prevalence of about 2-8% in the general population and an Increased prevalence of 14 - 28% in adults over the age of 60 (Finch and Warshaw, Dermatol. Ther., 2007, 20, 31-46).

The fungi infecting the nail include dermatophytes, moulds and yeasts (mainly from the Candida species). In over 90% of the cases, dermatophytes (especially Trichophyton rubrum) are the cause of the infection. There are different types of onychomycosis of the toenails (Seebacher et al.. Mycoses, 2007, 50(4), 321-327):
● *Distal-lateral subungual onchyomycosis (DLSO):* the most common form of fungal Infection of nails. In this case, the fungi invade the underside of the nail plate via the hyponychium and spread gradually to the matrix. Subungual hyperkeratosis and oncholysis develop, leading to yellow discoloration and a raised nail plate.
● *Proximal subungual onychomycosis (PSO):* the least common form In otherwise healthy people, but found more commonly in immunocompromised patients. In this case, the fungi proliferate distally within the nail plate starting at the matrix.
● Superficial white onychomycosis (SWO): accounts for only 10 percent of cases. This form Is characterized by chalky white patches on the surface of the nail plate.

Infections of the nails affect the quality of life of patients in terms of physical mobility and social embarrassment. Particularly in the case of thick toenails, the pressure may cause irritation and pain. Onychomycosis does not resolve spontaneously; it may worsen, spread to other uninfected locations (other nails or surrounding skin) or infect other people. Also, it may support additional bacterial infection. It is, therefore, important to find an effective treatment for this disease.

As a general rule, topical therapy is recommended if less than 50% of the nail is infected and the matrix is not involved. In all other cases, especially if the nail matrix is affected, systemic therapy (sometimes in combination with topical therapy) is currently recommended (Lecha et al., J. Eur. Acad. Dermatol. Venereol., 2005, 19(1), 25-33). Topical therapy of nail diseases has obvious advantages, such as elimination of systemic adverse events and of drug interactions and reduced cost of treatment. In general, however, evidence for the management of topical treatments for infections of the toenails is sparse. Available topical onychomycosis therapies Include ciclopirox olamine nail lacquer (8%) and amorolfine nail lacquer (5%).

Probably as a result of the poor drug penetration from these products through the nail, treatment times are long (at least one year) and cure rates low (6.5% to 12% cure rate for ciclopirox after 48 weeks (e.g. Baran R. et al. JEADV 2009).

Thus, alternative formulations are being sought, which improve drug delivery to the nail (ungual delivery) and result in shorter treatment times and higher cure rates than the available topical treatment options.

Terbinafine is one of the leading anti-fungal agent for oral treatment of onychomycosis. Administration in cream, gel, solution and spray dosage forms for use in topical treatment of fungal Infections has been approved in some countries, however, not for the treatment of onychomycosis.

Drug delivery to the nail is complicated due to the physical structure of human nails. The nail plate is much thicker, harder and more dense than the stratum corneum, which means a much longer diffusion pathway for drug delivery. This may lead to insufficient nail plate penetration and permeation, which explains the low efficacy rates of the topical nail antifungal formulations presently on the market.

Currently approved and experimental nail lacquers which are based on organic solvents, fail to provide nail hydration. They even remove water from the keratin matrix and, therefore, dry out the nail. Furthermore, due to the fact that the major solvents of these lacquers are ethanol and/or ethyl acetate, which evaporate very quickly, the formulations tend to dry out in minutes. This provides only a very short time for penetration of actives from the dissolved state. Additionally, lacquers typically form a film on the nail plate and do not aid to remove diseased nail material which often acts as a reservoir for spores and causes relapses of onychomycosis.

Creams are also used in the treatment of fungal infections of the skin. However, approved creams for amorolfine and terbinafine are formulations with a low drug load (0,25% and 1%, respectively), their nail hydration is only moderate and do not result in a removal of diseased nail material.

Therefore, there is a need for new formulations with a high drug load and which provide strong nail hydration, long penetration time, and allow the removal of diseased nail and spores, thereby resulting in superior clinical efficacy.

Formulations having high amounts of water are preferable since water seems to facilitate ungual permeation of certain molecules. However, a particular challenge for the development of such formulations Is to achieve high concentrations of antifungal agent in the presence of high amounts of water, since these drugs are known to have low water solubilites.

We have developed formulations with high contents of water and high concentrations of terbinafine. These formulations show increased permeation through the nails and allow for precise dosing and excellent adhesiveness to the nail plate.

### SUMMARY OF THE INVENTION

New topical pharmaceutical compositions have been developed comprising, based on the total weight of the composition:
a) from 1.5 wt. % to 5 wt. % of terbinafine or any pharmaceutically acceptable salt thereof;
b) from 15 wt. % to 35 wt. % of urea; and
c) more than 25 wt. % of water.

The invention further relates to a composition as defined above for use In the treatment or prevention of topical fungal infections, preferably onychomycosis.

### DETAILED DESCRIPTION OF THE INVENTION

Terbinafine is an allylamine antifungal drug that inhibits the growth of fungi by blocking the enzyme squalene epoxidase, a key enzyme in fungal ergosterol biosynthesis. Examples of other allylamines antifungal agents include amorolfine, naftifine and butenafine.

In one embodiment of the present invention, the amount of terbinafine or a pharmaceutically acceptable salt thereof is in the range of 2 wt. % to 4 wt. %. In a preferred embodiment, the amount of terbinafine is in the range of 2 wt. % to 3 wt. %.

Preferably, terbinafine is In the form of terbinafine hydrochloride.

In one embodiment of the present invention, the amount of urea is in the range of 15 wt. % to 30 wt. %. In a preferred embodiment, the amount of urea is in the range of 15 wt. % to 25 wt. %.

Typically, the amount of water is more than 35 wt. %, preferably more than 40 wt. % and more preferably more than 45 wt. %.

In one embodiment of the present invention, the amount of water is In the range of 25 wt.% to 65 wt.%, In a preferred embodiment, the amount of water is in the range of 35 wt.% to 55 wt,%. In a more preferred embodiment, the amount of water is in the range of 40 wt.% to 50 wt.%.

In a particular embodiment, the topical semi-solid pharmaceutical composition comprises, based on the total weight of the composition:
a) from 2 wt. % to 3 wt. % of terbinafine or a pharmaceutically acceptable salt thereof;
b) from 15 wt. % to 25 wt. % of urea; and
c) more than 40 wt. % of water.

In one embodiment of the present invention, the composition further comprises d) a carrier or vehicle selected from the group consisting of a C₂₋₆ alcohol, a C₂₋₈ polyol, a C₆₋₂₄ fatty acid triglyceride, a C₆₋₂₄ fatty alcohol, a C₆₋₂₄ alkyl sulfate, a C₆₋₂₄ fatty acid, a non-ionic surfactant or mixtures thereof.

In a preferred embodiment of the present invention, the carrier or vehicle is selected from the group consisting of a C₂₋₅ alcohol, a C₂₋₈ polyol and a non-ionic surfactant or mixtures thereof.

In a more preferred embodiment of the present invention, the carrier or vehicle is selected from the group consisting of a ethanol, propylene glycol and polysorbate 80 or mixtures thereof.

In a particular embodiment, the topical semi-solid pharmaceutical composition comprises, based on the total weight of the composition:
a) from 2 wt. % to 3 wt. % of terbinafine or a pharmaceutically acceptable salt thereof;
b) from 15 wt. % to 25 wt. % of urea;
c) more than 40 wt. % of water; and
d) a carrier or vehicle selected from the group consisting of ethanol, propylene glycol and polysorbate 80 or mixtures thereof.

In a preferred embodiment, the carrier or vehicle is selected from the group consisting of a C₂₋₅ alcohol and a C₂₋₆ polyol or mixtures thereof.

In a preferred embodiment, the carrier or vehicle is selected from the group consisting of a C₂₋₅ alcohol and a non-ionic surfactant or mixtures thereof.

In a preferred embodiment, the carrier or vehicle is selected from the group consisting of a C₂₋₈ polyol and a non-ionic surfactant or mixtures thereof.

In a preferred embodiment, the carrier or vehicle is a C₂₋₆ alcohol, more preferably ethanol.

In a preferred embodiment, the carrier or vehicle Is a C₂₋₈ polyol, more preferably propylene glycol.

In a preferred embodiment, the carrier or vehicle is a non-ionic surfactant, more preferably polysorbate 80.

In a further embodiment of the present invention, the composition further comprises e) a gelling agent.

In a preferred embodiment, the gelling agent is selected from the group consisting of hyaluronic acid or any pharmaceutically acceptable salt thereof and a polyquaternium polymer. In a more preferred embodiment, the gelling agent is sodium hyaluronate.

Typically, the amount of gelling agent is in the range of 0.5 wt. % to 5 wt. %, preferably from 1 wt. % to 3 wt. %.

In another embodiment of the present invention, the composition further comprises f) a buffering agent.

One embodiment of the invention relates to a topical pharmaceutical composition as defined above for use in the treatment or prevention of topical fungal infection, preferably onychomycosis.

Another embodiment of the invention relates to the use of a topical pharmaceutical composition as defined above for the manufacture of a medicament for the treatment or prevention of topical fungal infections, preferably onychomycosis.

A further embodiment of the invention relates to a method for treating a subject afflicted with a topical fungal infection which comprises applying to the affected area of skin or nail of said subject an effective amount of a composition as defined above.

The method of using the topical pharmaceutical composition of the invention is by applying it to completely cover the affected area. The usual frequency of application is once daily, although adequate maintenance therapy for some patients may be achieved with less frequent application.

Typically, the treatment should begin with trimming the infected nail or nails back as far as practical, followed by the use of a nail file to file the surface of the nail as thin as possible. Precautions should be taken to avoid filing or grinding through the nail into the skin.

In one embodiment of the present invention relates to a kit of parts comprising a composition as described above, a nail file and plasters, together with instructions for use.

In one embodiment, the present invention relates to the use of urea for solubilizing antifungal agents in topical formulations comprising more than 25% of water, based on the total weight of the composition.

In a preferred embodiment, the antifungal agents have a water solubility of less than 5 mg/ml and a log D of 2-3.5, measured at pH of 4 to 6.

In a preferred embodiment, the antifungal agent is selected from terbinafine, amorolfine and ciclopirox or any pharmaceutically acceptable salt thereof.

### Definitions

As used herein, the term C₂₋₅ alcohol embraces linear or branched alkyl groups having 2 to 5 carbon atoms, any of which may be substituted with one or more hydroxyl radicals.

Examples of C₂₋₅ alcohols include ethanol, propanol, butanol and pentanol.

As used herein, the term C₂₋₈ polyol embraces linear or branched alkyl groups having 2 to 8 carbon atoms, any of which may be substituted with at least two hydroxyl radicals. Examples of C₂₋₈ polyols include propylene glycol, butylene glycol, hexylene glycol and glycerol.

Suitable C₆₋₂₄ fatty acid triglycerides are normally found in animal and plant tissues, including those which have been hydrogenated to reduce or eliminate unsaturation, but can also be synthetically-prepared esters of glycerin and fatty acids. The fatty acids esterifying the different positions of glycerin can be different, giving rise to a large amount of possible combinations, including positional combinations. The position of the different fatty acids in natural triglycerides is not random, but rather it depends on the origin of the fat. The triglycerides more simple are those constituted by a sole fatty acid. Glyceryl esters of fatty acids can be advantageously chosen, for example, from the group consisting of synthetic, semi-synthetic and natural oils, as for example, animal fats and oils such as cow tallow, pig lard, bone oil, aquatic animal fats and oils (fish, such as herring, cod or sardine; cetaceans; etc.); and vegetable fats and oils such as avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its by-products such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

Suitable C₆-C₂₄ fatty alcohols according to the present invention are C₆-C₂₄ alcohols from vegetable and animal fats and oils, such as 2-octyldodecanol, 2-ethylhexanoyl alcohol, arachidyl alcohol, behenyl alcohol, caprylic alcohol, caproyl alcohol, capric alcohol, castor oil alcohol, ceterayl alcohol, palmityl (cetyl) alcohol, coconut alcohol, cotton alcohol, decyl alcohol, elaldyl alcohol, erucyl alcohol, gadoleyl alcohol, isostearyl alcohol, lauryl alcohol, linoleyl alcohol, linseed alcohol, myrlstyl alcohol, olein alcohol, olive pomace alcohol, oleyl alcohol, olive alcohol, palm alcohol, palm kernel alcohol, palmitoyl alcohol, petroselinic alcohol, rapeseed alcohol, ricinoleyl alcohol, safflower alcohol, soy alcohol, stearyl alcohol, sunflower alcohol, tall oil alcohol, tallow alcohol, tridecyl alcohol, or technical grade mixtures thereof such as cetostearyl alcohol.

As used herein, the term suitable C₆₋₂₄ alkyl sulfate embraces linear or branched alkyls having 6 to 24 carbon atoms, any of which may be substituted with one or more sulfate radicals.

Suitable non-ionic surfactants include, for example, an ethoxylated polysorbate, polyethylene glycol, ethylene oxide/propylene oxide copolymer, and polyethoxylated castor oil. Ethoxylated polysorbates, include, for example, polysorbate-20 (Tween-20), polysorbate-40 (Tween-40) and polysorbate-80 (Tween-80).

Any pharmaceutically acceptable buffering agents can be used to adjust the pH of the topical pharmaceutical compositions according to the invention to be within the acceptable range for topical administration, preferably in the range of 2.0 to 6.0, more preferably in the range of 2.5 to 4.5. Typically, the pH-adjusting agent can be an acid, an acid salt, or mixtures thereof. Example of suitable acids are known to those of skill in the art and illustratively include acetic, citric, fumaric, hydrochloric, phosphoric, lactic and nitric acids and the like, and mixtures thereof.

As used herein, the term topical fungal infection refers to disorders of the nails or the skin caused by different type of fungi. Examples of these disorders are onychomycosis, tinea in different parts of the body ie tinea corporis, tinea pedis, tinea cruris tinea capitis tinea versicolor (pityriasis versicolor), pityrosporum folliculitis and candidiasis (moniliasis). Preferably, the term topical fungal infection refers to onychomycosis.

The topical pharmaceutical composition according to the invention can be formulated in the form of a cream, a gel, a suspension, a lotion, a foam, a spray, an aerosol or a solution, preferably in the form of a semi-solid formulation such as a cream or a gel, more preferably in the form of a gel.

The Food and Drug Administration (FDA), Center for Drug Evaluation and Research (CDER) Data Standards Manual, Dosage Form (version 08) defines cream as "an emulsion, semisolid dosage form, usually containing more than 20% water and volatiles and/or less than 50% hydrocarbons, waxes, or polyols as the vehicle, which is generally for external application to the skin or mucous membranes". Gel is defined therein as "a semisolid dosage form that contains a gelling agent to provide stiffness to a solution or a colloidal dispersion".

The viscosity of the topical pharmaceutical composition of the invention will depend on the form of the composition. For Instance, in the case of a cream, the viscosity is typically in the range of 2,000 to 15,000 mPa.s, preferably in the range of 2,500 to 10,000 mPa.s, more preferably in the range of 3,000 to 7,000 mPa.s measured at 20°C using a DIN-Rotations Rheometer (Paar Physica); Measuring System Z 3 DIN ; D= 57 1/s.

In the case of a gel, the viscosity is typically in the range of 300 to 15000 mPa.s, preferably in the range of 500 to 12000 mPa.s, more preferably in the range of 1000 to 10000 mPa.s measured at 20°C using a DIN-Rotations Rheometer (Paar Physica): Measuring System Z 3 DIN; D= 57.2/s.

The topical pharmaceutical compositions according to the invention may optionally further comprise other well-known pharmaceutically and/or cosmetically acceptable additives, such as, e.g. anti-irritants, antioxidants, chelating agents, emollients, penetration enhancing agents, preservative agents, solubilizing agents, thickening agents, wetting agents, and the like, or mixtures thereof.

Examples of suitable anti-irritants are aloe vera, chamomile, alpha-bisabolol, cola nitida extract, green tea extract, tea tree oil, licoric extract, batyl alcohol (α-octadecyl glyceryl ether), selachyl alcohol (α-9-octadecenyl glyceryl ether), chimyl alcohol (α-hexadecyl glyceryl ether), panthenol, allantoin, caffeine or other xanthines, glycyrrhizic acid and derivatives thereof, and mixtures thereof.

Antioxidants used can be any antioxidants which are suitable or customary for cosmetic and/or dermatological applications. Suitable antioxidants are advantageously selected from the group consisting of amino acids (for example glycine, histidine, tyrosine, tryptophan) and derivatives thereof, imidazoles (e.g. urocanic acid) and derivatives thereof, peptides such as D,L-carnosine, D-carnosine, L-carnosine and derivatives thereof (e.g. anserine), carotenoids, carotenes (e.g. α-carotene, β-carotene, lycopene) and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts) and sulphoximine compounds (e.g. buthionine sulphoximines, homocysteine sulphoximine, buthionine sulphones, penta-, hexa-, heptathionine sulphoximine) in very small tolerated doses (e.g. pmol to µmol/kg), also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), and coniferylbenzoate of benzoin, rutinic acid and derivatives thereof, ferulic acid and derivatives thereof, butylated hydroxytoluene, butylated hydroxyanisole, nordihydroguaiac resin acid, nordihydrogualaretic acid, trihydroxybutyrophenone, uric acid and derivatives thereof, mannose and derivatives thereof, zinc and derivatives thereof (e.g. ZnO, ZnSO₄), selenium and derivatives thereof (e.g. selenium methionine), stilbenes and derivatives thereof (e.g. stilbene oxide, trans-stilbene oxide) and the derivatives (salts, esters, ethers, sugars, nucleotides, nucleosides, peptides and lipids) of said active ingredients which are suitable according to the invention.

Suitable emollients, which can be used in the composition of the present invention include, for example, dodecane, squalane, cholesterol, isohexadecane, isononyl isononanoate, PPG ethers, petrolatum, lanolin, safflower oil, castor oil, coconut oil, cottonseed oil, palm kernel oil, palm oil, peanut oil, soybean oil, polyol carboxylic acid esters, derivatives thereof, and the like, and combinations thereof.

Examples of suitable penetration enhancing agents can include, e.g., dimethylsulfoxide (DMSO), N-methyl pyrrolidine, dimethyl formamide (DMF), allantoin, urazole, N,N-dimethylacetamide (DMA), decylmethylsulfoxide, polyethylene glycol monolaurate, propylene glycol, propylene glycol monolaurate, glycerol monolaurate, lecithin, the 1-substituted azacycloheptan-2-ones, particularly 1-n-dodecylcyclazacycloheptan-2-one, alcohols, glycerin, hyaluronic acid, transcutol, and the like, and combinations thereof. Certain oil components (e.g., certain vegetable oils such as, e.g., safflower oil, cottonseed oil and corn oil) also can exhibit penetration enhancing properties.

Examples of suitable preservatives to prevent microbial contamination are alkylparabens, particularly methylparaben, propylparaben and butylparaben; sodium benzoate; butylated hydroxy toluene; butylated hydroxyanisole; ethylenediamine tetraacetic acid; chlorobutanol; benzyl alcohol; phenylethylalcohol; dehydroacetic acid; sorbic acid; potassium sorbate; benzalkonium chloride; benzathonium chloride; and mixtures thereof. The amount of preservative generally utilized will vary depending upon the preservative selected.

Examples of further solubilizing agents are, for example, nonionic surfactants from at least one of the following groups: products of the addition of 1 to 30 moles of ethylene oxide and/or 0 to 5 moles of propylene oxide onto linear C₈-C₂₂ fatty alcohols, C₁₂-C₂₂ fatty acids and alkyl phenols containing 8 to 15 carbons in the alkyl group; alkyl and/or alkenyl oligoglycosides containing 8 to 22 carbons in the alkyl group and ethoxylated analogs thereof; addition products of 1 to 15 moles of ethylene oxide with castor oil and/or hydrogenated castor oil; addition products of 15 to 60 moles of ethylene oxide with castor oil and/or hydrogenated castor oil; partial esters of glycerol and/or sorbitan with unsaturated or saturated, linear or branched fatty acids containing 12 to 22 carbons and/or hydroxycarboxylic acids containing 3 to 18 carbon atoms and addition products thereof with 1 to 30 moles of ethylene oxide; mixtures of alkoxylated glycerides and alkoxylated glycerine, partial esters of polyglycerol (average degree of selfcondensation 2 to 8), polyethylene glycol (weight average molecular weight 400 to 5000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbons and/or hydroxycarboxylic acids containing 3 to 18 carbons and addition products thereof with 1 to 30 moles of ethylene oxide; mixed esters of pentaerythritol, fatty acids, citric acid and fatty alcohol and/or mixed esters of fatty acids containing 6 to 22 carbons, methyl glucose and polyols, preferably glycerol or polyglycerol; mono-, di- and trialkyl phosphates and mono-, di-and/or tri-PEG-alkyl phosphates and salts thereof; block copolymers, for example Polyethyleneglycol-30 Dipolyhydroxystearate; polymer emulsifiers; polyalkylene glycols and alkyl glyceryl ethers. Particularly preferred solubilizing agents are products of the addition of 1 to 30 moles of ethylene oxide and/or 0 to 5 moles of propylene oxide onto linear C₈-C₂₂ fatty alcohols such as lauryl, myristyl, cetyl (palmityl), stearyl, oleyl, and ricinoleyl alcohols, or technical grade mixtures thereof such as cetostearyl alcohol or palmitoleyl alcohol.

A thickening agent or viscosity-enhancing agent can be included to generally thicken the liquid pharmaceutical compositions. While any suitable thickening agent can be included in the compositions of the present invention, a preferred thickening agent, when used, includes one or more of acacia, alginic acid bentonite, carbomer, carboxymethylcellulose calcium or sodium, cetostearyl alcohol, methyl cellulose, ethylcellulose, glycerin, gelatin guar gum, hydroxyethylcellulose, hydroxypropylcellulose, hydroxypropylmethylcellulose, maltodextrin, polyvinyl alcohol, povidone, propylene carbonate, propylene glycol alginate, sodium alginate, sodium starch glycolate, starch tragacanth, and xanthan gum, and any combination thereof. More preferred thickening agents are glycerin, hydroxypropylmethylcellulose, and xanthan gum, and any combination thereof.

Examples of wetting agents (chemical substances that increase the spreading and penetrating properties of a liquid by lowering its surface tension) include one or more cationic surfactants, such as benzalkonium chloride; non-ionic surfactants such as polyoxyethylene and polyoxypropylene block copolymers; polyoxyethylene fatty acid glycerides and oils (such as polyoxyethylene (6) caprylic/capric mono- and diglycerides), polyoxyethylene (40) hydrogenated castor oil; polyoxyethylene sorbitan esters, such as polysorbate 20 and polysorbate 80; propylene glycol fatty acid esters, such as propylene glycol laureate; glyceryl fatty acid esters, such as glyceryl monostearate; sorbitan esters, such as sorbitan monolaurate, sorbitan monooleate, sorbitan monopalmitate and sorbitan monostearate; glyceryl fatty acid esters, for example glyceryl monostearate; anionic surfactants such as sodium lauryl sulphate, sodium lauryl ether sulphate; or fatty acids and salts thereof, such as oleic acid, sodium oleate and triethanolamine oleate.

The following examples are given in order to provide a person skilled in the art with a sufficiently clear and complete explanation of the present invention, but should not be considered as limiting of the essential aspects of its subject, as set out in the preceding portions of this description.

### EXAMPLES

### Examples 1-12

Compositions according to the invention were prepared as indicated in Table 1 (wt.% based on the total weight of the composition)

**Table 1:**

| **Example** | 1 | 2 | 3 | 4 | 5 | 6 | 7 | 8 | 9 | 10 | 11 | 12 |
|---|---|---|---|---|---|---|---|---|---|---|---|---|
| Terbinafine HCl | 2.5 | 2 | 2.5 | 1.5 | 2.5 | 2 | 3.5 | 2.5 | 4 | 2.5 | 2.5 | 2 |
| Urea | 20 | 20 | 20 | 20 | 20 | 15 | 35 | 35 | 20 | 20 | 20 | 20 |
| Polysorbate 80 | 8.5 | 8 | 8 | 8.5 | 8.5 | 8.5 | 8.5 | 8.5 | 8.5 | 8.5 | 8.5 | 8.5 |
| Propylene glycol | 15 | 12 | 7 | 7 | 7 | 7 | 7 | 7 | 15 | 7 | 7 | 7 |
| Ethanol | - | - | 7 | 7 | 7 | 7 | 7 | 7 | 15 | 7 | 7 | 7 |
| Water | 49 | 53 | 50.5 | 51.5 | 51 | 56 | 34.5 | 35.5 | 33 | 48.13 | 47.64 | 48.14 |
| Sodium hyaluronate | 2 | 2 | 2 | 2 | 2 | 2 | 2 | 2 | 2 | 1.5 | 1.8 | 1.8 |
| | pH adjusted to 3.5-4.5 | | | | | | | | | pH adjusted to 2.5-3.5 | | |

Said compositions were prepared in the following manner:
1) Urea, polysorbate 80, propylene glycol, ethanol, water and the pH adjusting agents were mixed.
2) The Ingredients were heated to 60°C and dissolved while stirring, until a homogenous and clear solution was obtained.
3) Terbinafine was added to the mixture until dissolved.
4) Sodium hyaluronate was transferred to the batch while homogenizing.
5) The batch was cooled down to 30°C under mild stirring.

### Examples 13-14

Compositions according to the invention were prepared as Indicated in Table 2 (wt.% based on the total weight of the composition).

**Table 2:**

| **Example** | 13 | 14 |
|---|---|---|
| Terbinafine HCl | 3 | 3 |
| Urea | 20 | 20 |
| Cetearyl alcohol | 1.2 | 1.2 |
| Cetearyl sulphate | 1.8 | 1.8 |
| Stearic add | 7 | 7 |
| Mid chain triglycerides | 18 | 18 |
| Polysorbate 80 | 7 | 7 |
| Propylene glycol | 10 | 10 |
| Purified water | 28.2 | 28.5 |
| Sodium hyaluronate | 0.8 | - |
| Xanthane | - | 0.5 |
| | pH adjusted to 3.5-4.5 | |

Said compositions were prepared in the following manner:
1) Cetearyl alcohol, stearic acid, mid-chain triglycerides were added to a stainless steel container. The ingredients were heated to 70°C and melted while stirring (oil phase).
2) Ceterayl sulphate, urea, polysorbate 80, propylene glycol, water and the pH adjusting agents were mixed and heated at 70°C. Then terbinafine was added until complete dissolution (water phase).
3) Oil and water phases were mixed under homogenization.
4) Sodium hyaluronate was transferred to the batch while homogenizing.
5) The batch was cooled down to 30°C under gentle stirring.

These formulations show a good penetration Into and permeation through the nails and allow for precise dosing and excellent adhesiveness to the nail plate.

Modifications, which do not affect, alter, change or modify the essential aspects of the pharmaceutical compositions described, are included within the scope of the present invention.

## Claims

1. A topical pharmaceutical composition comprising, based on the total weight of the composition:
a) from 1.5 wt. % to 5 wt. % of terbinafine or any pharmaceutically acceptable salt thereof;
b) from 15 wt. % to 35 wt. % of urea; and
c) more than 25 wt. % of water.

2. A composition according to claim 1, wherein the amount of terbinafine or a pharmaceutically acceptable salt thereof is in the range of 2 wt. % to 4 wt. %, preferably of 2 wt, % to 3 wt. %.

3. A composition according to any preceding claim, wherein the amount of urea is in the range of 15 wt. % to 30 wt. %, preferably of 15 wt. % to 25 wt. %.

4. A composition according to any preceding claim, wherein the amount of water is more than 35 wt. %, preferably more than 40 wt. %.

5. A composition according to any preceding claim:
a) from 2 wt. % to 3 wt. % of terbinafine or a pharmaceutically acceptable salt thereof;
b) from 15 wt. % to 25 wt. % of urea; and
c) more than 40 wt. % of water.

6. A composition according to any preceding claim further comprising d) a carrier or vehicle selected from the group consisting of a C₂₋₅ alcohol, a C₂₋₈ polyol, a C₆₋₂₄ fatty acid triglyceride, a C₆₋₂₄ fatty alcohol, a C₆₋₂₄ alkyl sulfate, a C₆₋₂₄ fatty acid, a non-ionic surfactant or mixtures thereof.

7. A composition according to claim 6 wherein the carrier or vehicle is selected from the group consisting of a C₂₋₅ alcohol, a C₂₋₈ polyol and a non-ionic surfactant or mixtures thereof.

8. A composition according to claim 7 wherein the carrier or vehicle is selected from the group consisting of a ethanol, propylene glycol and polysorbate 80 or mixtures thereof.

9. A composition according to any preceding claim, wherein the composition further comprises e) a gelling agent.

10. A composition according to claim 9, wherein the gelling agent is selected from the group consisting of hyaluronic acid or any pharmaceutically acceptable salt thereof and a polyquaternium polymer.

11. A composition according to claim 9 or 10, wherein the amount of gelling agent is in the range of 0.5 wt. % to 4 wt. %, preferably from 1 wt. % to 3 wt. %.

12. A composition according to any preceding claim, further comprising f) a buffering agent.

13. A composition according to any one of claims 1 to 12 for use in the treatment of topical fungal infections, preferably onychomycosis.

14. Use of a composition as defined in any on of claims 1 to 12 in the manufacture of a medicament for the topical treatment of fungal infection, preferably onychomycosis.

15. A method for treating a subject afflicted with a topical fungal infection, preferably onychomycosis, which comprises applying to said subject an effective amount of a composition as defined In any one of claims 1 to 12.

16. A kit of parts comprising a composition according to claim 1, a nail file and plasters, together with instructions for use.

17. Use of urea for solubilizing antifungal agents In topical formulations comprising more than 25% of water, based on the total weight of the composition.

18. Use according to claim 17 wherein the antifungal agent is selected from terbinafine, amorolfine and ciclopirox, or any pharmaceutically acceptable salt thereof.
